# EUROPEAN PATENT APPLICATION

(11) **EP 4 703 009 A1**
(43) Date of publication of application: **04.03.2026**
(21) Application number: 24198285.9
(22) Date of filing: 03.09.2024
(51) Int. Cl.: A61N 5/06

(54) **POSITIONING DEVICE FOR POSITIONING A LIGHT-CONDUCTING FIBER IN A CALIBRATION PORT**

(71) Applicant: Omicron-Laserage Laserprodukte GmbH, 63110 Rodgau-Dudenhofen (DE)
(72) Inventor: BAUMANN, Sönke-Nils, 63739 Aschaffenburg (DE)
(74) Representative: Patentanwälte Olbricht Buchhold Keulertz

(57) **Abstract**

The invention relates to a positioning apparatus (100) for positioning a light-guiding fiber (203), wherein the positioning apparatus (100) comprises a main body (101) having a base portion (102) and an elongate portion (103), wherein the base portion (102) of the main body (101) has a first end face (104) and wherein the elongate portion (103) of the main body (101) has a second end face (105), and wherein the main body (101) comprises a channel (106) for receiving the light-guiding fiber (203), the channel (106) extending along a longitudinal axis (A) of the main body (101) from the first end face (104) through the base portion (102) and at least partly through the elongate portion (103). The positioning apparatus (100) is characterized in that the positioning apparatus (100) is configured to transmit light from a light-guiding fiber (203) to a sensor means without the use of an emission opening in the elongate portion (103) of the main body (101). A further aspect of the invention relates to a system (200) comprising said apparatus (100).

## Description

The invention relates to a positioning apparatus for positioning a light-guiding fiber in a calibration port of a medical apparatus, to a system with a positioning apparatus, at least one light source, and at least one light-guiding fiber, and to a method for calibrating a light source of said system.

Laser radiation is well-known in medical technology. In addition to the established applications, such as, surgery or ophthalmology, the coherent monochromatic radiation of a laser source is increasingly used in for activation of corresponding drugs or medicaments. A prominent example in this respect is photodynamic therapy (PDT).

As part of PDT, a patient is administered a drug that mainly attaches itself to tumor cells or bacteria. By absorbing electromagnetic radiation of a certain wavelength, the drug is activated, thus causing it to have its desired medical effect on the cells to which it is attached. This is novel way to combat tumors, or to visualize areas of the human body that are affected by certain bacteria.

In order to expose corresponding areas of the human body to electromagnetic radiation or laser radiation, it is known in the prior art to couple corresponding laser radiation into a light-conducting fiber and to couple it out of the light-conducting fiber at the treatment site, such that the target area is exposed to the light. It is crucial, however, that the target area is exposed to a certain radiance (power per area) of the laser, as effective treatment may otherwise not be possible. If, for example, the applied radiance is too low, the drug may not be activated. If the radiance is too high, the treated tissue can be subject to injuries caused by irradiation.

To avoid the above drawbacks, the light power (radiant flux) that is coupled out of a light-conducting fiber is usually calibrated before treatment. For example, the light-conducting fiber may be placed in an integrating sphere, such that the total light power emitted by the fiber is recorded. However, since the fiber will subsequently be used for a medical procedure, sterility of the light-conducting fiber during calibration must be guaranteed. The light-guiding fiber may therefore be surrounded by a sterile sleeve, which serves as a barrier between the sterile fiber and the non-sterile measuring device.

A method for calibrating a light-guiding fiber is known DE 10 2017 112 482 A1. The method comprises a positioning apparatus for positioning the fiber in a calibration port of a medical apparatus. Once the positioning apparatus is inserted into the calibration port and the light-guiding fiber is inserted into the positioning apparatus, radiation coupled out of the fiber emerges through one or more cutouts of the positioning apparatus and is subsequently determined by appropriate sensors of the calibration port, e.g. by photodiodes, photoresistors, phototransistors, and/or photovoltaic light sensors.

However, some fibers are configured to specifically emit light in a specific direction lateral to the fiber. Some fibers may for example emit light in a 90° angle relative to the direction in which the light is guiding through the fiber, i.e. in a direction perpendicular to the light-guiding fiber. In order to detect the light emitted and to calibrate the fiber, it is important that the light can pass through the positioning apparatus. This is usually accomplished by one or more lateral cutouts in the body of the positioning apparatus.

The objective technical problem of the present invention is to present an alternative to the known solutions by providing an improved positioning apparatus for positioning and aligning a light-guiding fiber in a calibration port.

The problem is solved by a positioning apparatus for positioning a light-guiding fiber in a calibration port of a medical apparatus in accordance with claim 1, and by a system comprising said positioning apparatus in accordance with claim 13. Special embodiments or beneficial variants of the apparatus are presented in claims 2 to 12. Accordingly, special embodiments or beneficial variants of the system are presented in claims 14 to 20.

The positioning apparatus for positioning a light-guiding fiber comprises a main body having a base portion and an elongate portion, wherein the base portion of the main body has a first end face and wherein the elongate portion of the main body has a second end face, and wherein the main body comprises a channel for receiving the light-guiding fiber, the channel extending along a longitudinal axis of the main body from the first end face through the base portion and at least partly through the elongate portion, characterized in that the positioning apparatus is configured to transmit light from a light-guiding fiber to a sensor means without the use of an emission opening in the elongate portion of the main body. In particular, the positioning apparatus is configured to transmit light from a light-guiding fiber to a sensor means in a radial direction without the use of an emission opening in the elongate portion of the main body without the use of a lateral emission opening.

By allowing light leaving the light-guiding fiber to pass through the elongate portion of the main body, user of the respective calibration system can easily ascertain that the light coupled out of the light-guiding fiber at hits the sensors of the medical apparatus, thereby eliminating the risk that only a portion of the desired amount of light emitted from the fiber hits the sensors, or even that no light hits the sensors at all. The elongate portion does not require any openings or cutouts in order to allow light to pass, neither along its sides nor at its end face. This means that the manufacturing process of the positioning apparatus can be simplified, as the step of providing emission openings can be omitted, thus minimizing production costs.

According to another embodiment, the elongate portion of the main body or a portion thereof may be comprised of a semitransparent material. According to another embodiment, wherein the elongate portion of the main body or a portion thereof is coated with a semitransparent material. According to yet another embodiment, the elongate portion of the main body or a portion thereof may be comprised of a fully transparent material. In particular, at least 50 % of the elongate portion may comprise a semitransparent material and/or may be coated with a semitransparent material, or may comprise a fully transparent material. According to this disclosure, "transparent" or "transparency" refers to the physical property of allowing the light from the light-guiding fiber to pass through the material with no or only very low levels of scattering or absorption of said light. Therefore, in accordance with this disclosure, a "transparent" material is understood to be "not opaque" with respect to the radiation emitting from the light-guiding fiber. Accordingly, "semitransparent" may be understood to mean that a given material may absorb or scatter some portion of the light passing through (but not all of the light).

According to another embodiment, at least a portion of the main body may comprise and/or may be coated with a sterilizable material. For example, the channel of the positioning apparatus and the regions of the body of the positioning apparatus adjoining said channel, in particular, are preferably sterilizable. Furthermore, the entire body of the positioning apparatus may consist of a sterilizable material.

According to another embodiment, the sterilizable material may comprise a plastic, in particular polyoxymethylene. The sterilizable material should also be transparent or at least semitransparent for the light coupled out of the fiber in a radial direction.

According to another embodiment, the main body may be rotationally symmetric about the longitudinal axis of the main body. This allows for the positioning apparatus to be inserted into the respective port of the medical apparatus with any rotational orientation, thereby providing a degree of freedom and thus facilitating insertion of the positioning apparatus.

According to another embodiment, base portion of the main body may comprise at least one control element for ascertaining the rotational orientation of the light-guiding fiber along the longitudinal axis when the light-guiding fiber is received by the channel. With the control element, insertion of the positioning apparatus into the medical apparatus in a desired rotational orientation can be achieved. This guarantees that light coupled out of the light-guiding fiber in a radial direction hits a respective sensor of the medical apparatus.

According to another embodiment, the at least one control element may comprise one or more of a groove in the first end face, a non-symmetric feature of the positioning apparatus, a text on the outer surface of the positioning apparatus and a symbol on the outer surface of the positioning apparatus. This way, the control element is easily visible for the user. Furthermore, a groove allows the user to also track at least part of the light-guiding fiber while it is inserted into the positioning apparatus. Therefore, should the fiber have orientational markings as well, a user may easily make sure that the marking of the fiber and the control element of the positioning apparatus are aligned to each other, thus ensuring that the light coupled out of the fiber at a certain angle hits the sensors as intended. A physical mark, such as a groove, also increases durability of the positioning apparatus, since the groove cannot fade away or otherwise diminish over time

According to another embodiment, the base portion may have a greater radius than the elongate portion of the main body. This way, the main body will abut to the medical apparatus when the positioning apparatus is fully inserted, ensuring the right insertion depth of the positioning apparatus within the medical apparatus.

According to another embodiment, the channel does not penetrate the second end face of the main body. In other words, the channel may end in the elongate portion of the main body. Since the position apparatus may configured for emission of light without an emission opening, and since light-guiding fiber may also be configured for light emission in a radial direction, e.g. from a specific radial emission point of the fiber, it may be beneficial to assure that the light-guiding fiber can only be inserted into the positioning apparatus at a specific depth, which is why a defined ending of the channel in in the elongate portion of the main body may be advantageous.

According to another embodiment, wherein the channel may comprise a main section and an end section, wherein the main section may comprise a first diameter D1 greater than the diameter of the light-guiding fiber and wherein the end section comprises a second diameter D2 that is less than the diameter of the light-guiding fiber. This way, it is possible to insert a light-guiding fiber that has diameter smaller than D1 and larger than D2 into the channel, such that the light-guiding fiber will abut against the end section of the channel, as it cannot enter the end section. Thus, the light-guiding fiber cannot be involuntarily inserted too deep into positioning apparatus. The end section allows light to pass through the channel in a longitudinal direction, which may be beneficial for embodiments of the positioning apparatus comprising only semitransparent material. In those cases, the light emitted from the fiber in the longitudinal direction will not be scattered by any material of the positioning apparatus, before it exits the positioning apparatus at the second end face of the elongate portion.

According to another embodiment, the main body may have a frontal cutout on the second end face of the elongate portion, wherein the frontal cutout is connected to the end section of the channel. This allows for a radiant flux output coupled out of the fiber in a frontal direction (i.e. from the tip of the light-guiding fiber) to emerge from the positioning apparatus. In this case, the frontal cutout may function an emission opening, meaning that only light that is coupled out of the fiber in radial direction is transmitted to a sensor means without the use of an emission opening in the elongate portion of the main body.

According to another embodiment, the diameter of the frontal cutout may increase in the direction of the second end face of the main body. The minimum diameter of the frontal cutout is preferably chosen in such a way that the fiber in the positioning apparatus is only supported at the cladding of the fiber, while the radiation-guiding core of the fiber is completely exposed by the cutout in the longitudinal direction. In any way, the minimum diameter of the frontal cutout should always be smaller than the diameter of the light-guiding fiber at its tip. This prevents fiber from being pushed out of the positioning apparatus through the frontal cutout.

Another aspect of the invention relates to a system comprising the positioning apparatus as described herein, and a medical apparatus, wherein the medical apparatus comprises at least one light source, a calibration port, and the sensor means, and wherein the positioning apparatus is configured to position the light-guiding fiber in the calibration port of the medical apparatus.

The light source generates laser radiation having a specific radiant flux and a specific wavelength. By way of example, the light source can be a laser source, for example one or more laser diodes. The laser diodes may be connected to the light-guiding fiber by way of an appropriate optical unit such that the radiant flux or laser radiation emitted by the laser diode is coupled into the light-guiding fiber. By way of example, an optical fiber with a thickness of 200-1200 µm, preferably of 400-600 µm, can be used as a light-guiding fiber. Here, typical radiant fluxes used in the field of 20 PDT lie in the range of 0.25-10 watt, preferably in the range of 1.5-5 watt.

According to another embodiment, the medical apparatus may comprise at least one lateral sensor arranged laterally at the calibration port, the lateral sensor preferably being a photodiode. This way, light coupled out of the light-guiding fiber in a lateral or radial direction can be determined by the medical apparatus.

According to another embodiment, the medical apparatus may comprise at least one frontal photodiode arranged at a longitudinal end of the calibration port. This way, light coupled out of the light-guiding fiber in a longitudinal direction can be determined by the medical apparatus.

According to another embodiment, the medical apparatus further may comprise at least one light-guiding fiber, the light-guiding fiber preferably being configured to emit light from lateral direction of the length of the fiber. According to another embodiment, the light-guiding fiber is positioned in the calibration port and wherein light emitted by the light-guiding fiber can at least partly emerge from the positioning apparatus to the sensor means. The light-guiding fiber may comprise a visual marker for tracing the rotational orientation of the light-guiding fiber when the light-guiding fiber is inserted in the channel of the positioning apparatus. The visual marker facilitates correct alignment of the light-guiding fiber with the control element of the positioning apparatus, thereby assuring correct alignment of the fiber in the medical apparatus, such that the light coupled out from the fiber in a lateral or radial direction can hit the sensor means of the medical apparatus. The visual marker of the light-guiding fiber may for example comprise a first strip printed or otherwise disposed in a longitudinal direction of the fiber and/or second strip in circumferential direction around the fiber.

According to another embodiment, the at least one calibration port may be configured to receive the positioning apparatus in such a way that, by way of the subsequent insertion of the at least one light-guiding fiber into the positioning apparatus, the light-guiding fiber is positioned relative to the sensor means such that the radiant flux of the laser radiation emerging from the light-guiding fiber can be determined by the sensor means.

According to another embodiment, the channel may further comprise a frontal cutout on the second end face configured to transmit light from the light-guiding fiber to a second sensor means, and wherein the at least one calibration port is further configured to receive the positioning apparatus in such a way that, by way of the subsequent insertion of a light-guiding fiber emitting light from the frontal end of the light-guiding fiber into the positioning apparatus, the light-guiding fiber is positioned relative to the sensor means such that the radiant flux of the laser radiation emerging from the light-guiding fiber can be determined by second sensor means that receives light transmitted from the light-guiding fiber to the second end face.

Any one of the embodiments, examples or features disclosed herein may be used in combination or separately and in conjunction with any one of the aspects of the disclosed subject matter, mutatis mutandis.

The accompanying drawings illustrate several examples of the subject matter described above. The drawings depict the following:

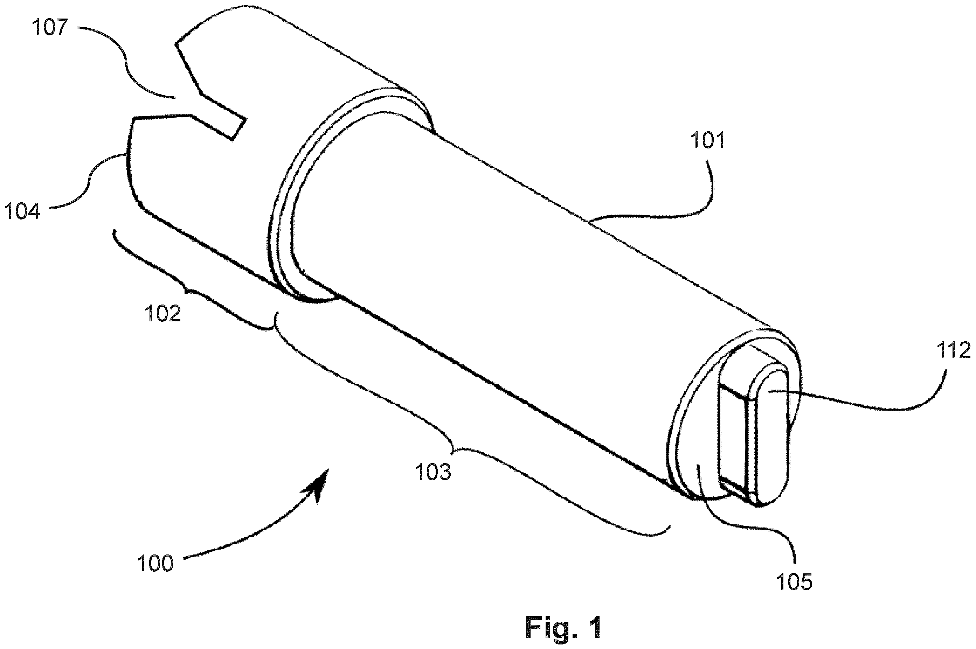

- Fig. 1: shows an isometric view of a positioning apparatus for positioning a light-guiding fiber in a calibration port of a medical apparatus;
- Fig. 1: shows an isometric view of another positioning apparatus for positioning a light-guiding fiber in a calibration port of a medical apparatus;
- Fig. 3: shows a lateral view of a positioning apparatus for positioning a light-guiding fiber in a calibration port of a medical apparatus;
- Fig. 4: shows a schematic illustration of a system comprising a medical apparatus and a positioning apparatus;
- Fig. 5: shows a schematic cross-sectional illustration of a system comprising a medical apparatus and a positioning apparatus.

Fig. 1 shows an isometric view of a positioning apparatus 100 for positioning a light-guiding fiber 203 in a calibration port 204 of a medical apparatus 201. The positioning apparatus 100 shown in Fig. 1 has a substantially rotationally symmetric main body 101 that encloses a channel 106 for a light-guiding fiber (not illustrated). The main body 101 has a base portion 102 and an elongate portion 103, which are also both substantially rotationally symmetric.

The base portion 102 of the main body 101 has a first end face 104. Accordingly, the elongate portion 103 of the main body 101 has a second end face 105. The main body 101 comprises a channel 106 which, in case of the embodiment shown in Fig. 1, extends from the first end face 104 through to the second end face 105 along a longitudinal axis A of the main body 101.

The diameters of the base portion 102 and the elongate portion 103 differ from each other. Furthermore, the base portion 102 extends from the first end face 104 over approximately one quarter of the total length of the positioning apparatus 100, while the elongate body 103 extends from a second end face 105 over the remaining length of the positioning apparatus 100 (i.e., approximately three-quarters). The edges of the base portion 102 and the elongate portion 103 of the main body 101 are preferably beveled by a chamfer such that there are no sharp edges on the positioning apparatus 100.

As an exception to the rotationally symmetry, the base portion 102 of the positioning apparatus 100 shown in Fig. 1 comprises a control element 107 in the form of a groove 114 formed into the first end face 104 of the main body 101. The groove 114 extends longitudinally across the first end face 104, essentially cleaving at least the surface of the first end face 104. As shown in Fig. 1, the groove 114 may comprise a funnel-shaped section 115, wherein the width of the funnel decreases in a direction towards the elongate body 103. However, the grove 114 is preferably only funnel-shaped when viewed from a direction along the grove's 114 progression across the surface of the first end face 104. The funnel-shaped section 115 of the groove 114 of Fig. 1 then transitions into a straight section 116, which ends blindly in the base portion 102, with the exception of the channel 106, which continues to protrude through the base portion 102 and the elongate portion 103.

Furthermore, an alignment element 112 is disposed on the second end face 105 of the positioning apparatus 100. In embodiment shown in Fig. 1, the alignment element 112 is an extrusion protruding outward from the second end face 105, said extrusion having rounded corners.

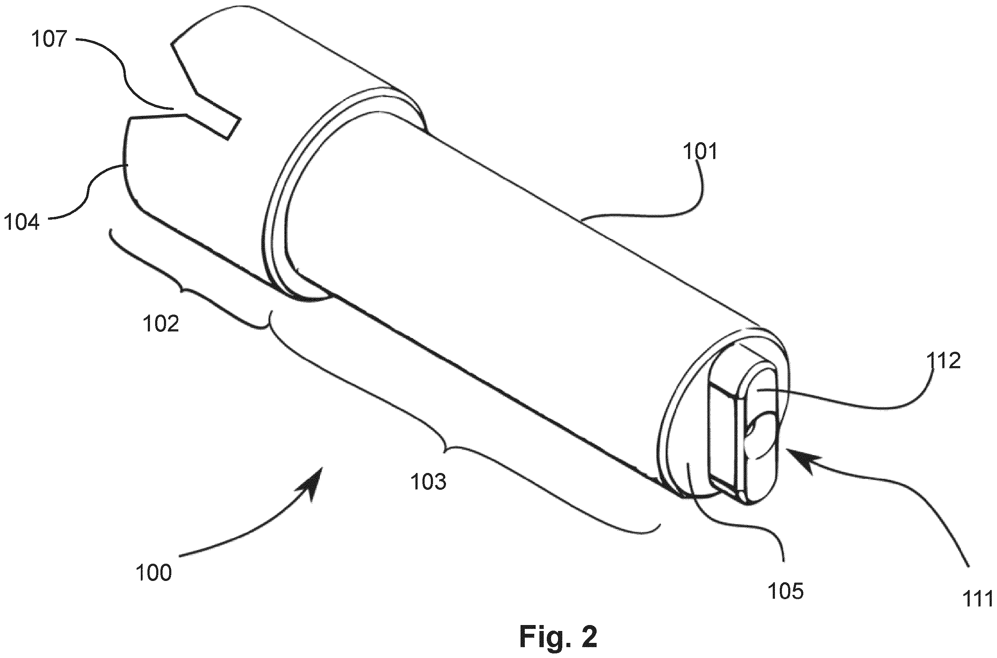

Fig. 2 shows an isometric view of another positioning apparatus 100 for positioning a light-guiding fiber 203 in a calibration port 204 of a medical apparatus 201. The positioning apparatus 100 shown in Fig. 2 differs from the embodiment of Fig. 1 in that the alignment element 112 comprises a frontal cutout 111 at the tip of the positioning apparatus 100, which may extend to the second end face 105 of the elongate portion 103. This way, if the channel 106 (not shown in Fig. 2) extends all the way through the elongate portion 103 to the end face 105, light can exit from the light-guiding fiber 203 in a longitudinal direction L through said frontal cutout 111.

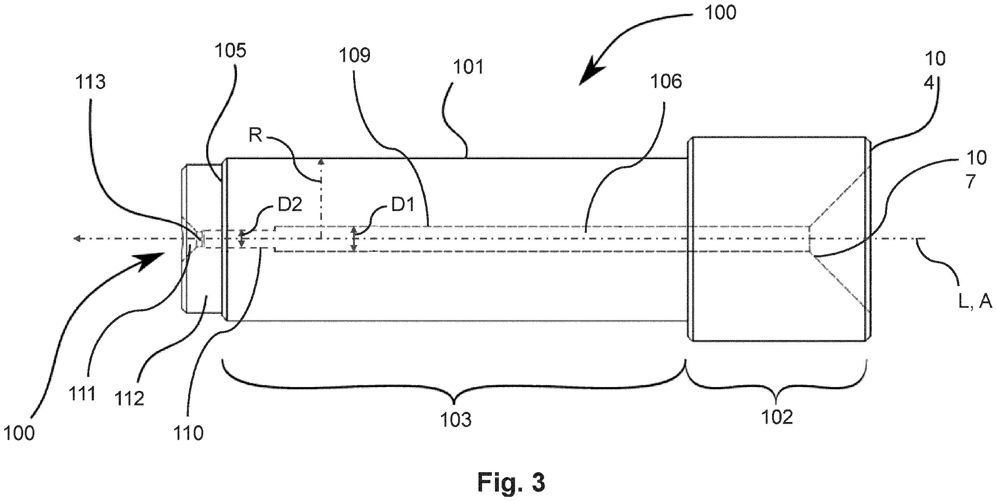

Fig. 3 depicts a lateral view of a positioning apparatus 100, with the grove 114 and the channel 106 being made visible in the illustration through broken lines. The channel 106 extends through the main body 101 along the longitudinal axis A of the positioning apparatus 100. As is shown in Fig. 3, the funnel-shaped section 115 of the grove 114 leads to the channel 104, which then extends through the main body 101 of the positioning apparatus 100 to the second end face 105.

As shown in Fig. 3, the channel 106 may comprise a main section 109 and an end section 110, wherein the main section 109 has a first diameter D1 that is larger than a second diameter D2 of the end section 110. The groove 114 in the first end face 104 of the positioning apparatus 100 transitions into the main section 109 of the channel 106. The main section 109 then progresses through the base portion 102 and through most of the elongate portion 103, before the diameter D1 of the main section 109 abruptly decreases to the diameter D2 of the end section 110. Ideally, the first diameter D2 is just large enough that a light-guided fiber 203 can be inserted in the main section 109 of channel 106 until it reaches the end section 110 of the channel 106 where it cannot enter due to the end section's smaller diameter D2. This prevents the light-guiding fiber 203 from being inserted too deep into positioning apparatus 100 and ensures that the fiber 203 is always inserted into the positioning apparatus 100 with the same depth, provided that its diameter is larger than that of the end section 110. However, a light-guiding fiber 203 having a maximum diameter that is smaller than both D1 and D2, e.g. a light-guiding fiber 203 that emits light at its frontal tip, can be still inserted all the way through the end section 110 of the channel 106 until it reaches an aperture 113.

In the example shown in Fig. 3, the channel 106 extends almost entirely through the positioning apparatus 100, i.e. from the groove 114 in the first end face 104, to the second end face 105, on which the alignment element 112 is disposed. However, the channel 106 does not penetrate the second end face 105. This means that light emitted from the fiber in the longitudinal direction L must only pass through a thin stretch of the positioning apparatus' 100 end face 105, which is preferably made from a transparent or at least semitransparent material. As further shown in Fig. 3, the alignment element 112 comprises a frontal cutout 111, which comprises a channel section having diameter that is similar to the second diameter D2 of the end section 110 of the cannel 106, an aperture 113 that is preferably adapted to the diameter of the core of a light-guiding fiber 203 inserted into the positioning apparatus 100, and a funnel-shaped section. In other words, the channel section of the frontal cutout 111 in the alignment element 112 subsequently widens in the longitudinal direction L.

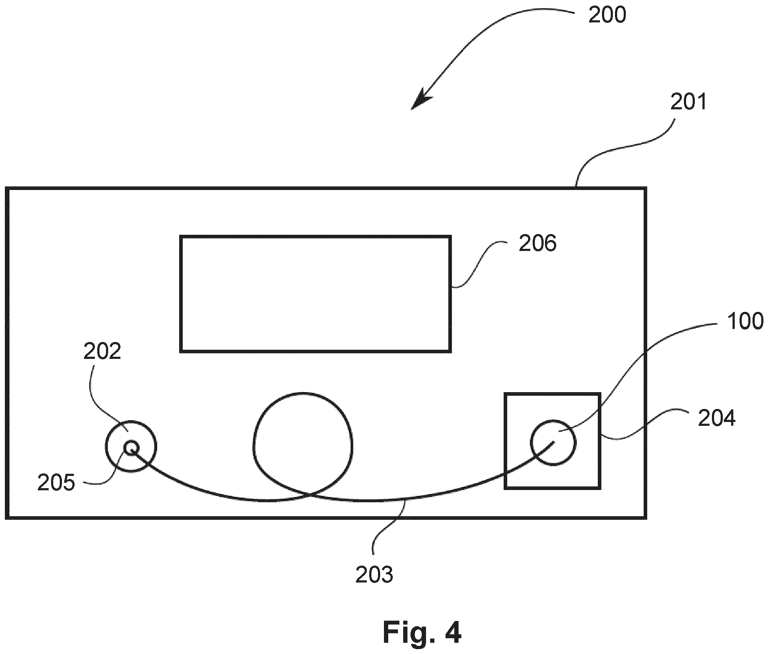

Fig. 4 shows a schematic illustration of a system 200 comprising a medical apparatus 201 and a positioning apparatus 100. The medical apparatus 201 comprises at least one light source 202, at least one light-guiding fiber 203 and at least one positioning apparatus 100 as presented herein. By way of example, the light source 202 can be one or more of a laser diode. The light-guiding fiber 203 is connected to the light source 202 of the medical apparatus 201 in such a way that at least a portion of laser radiation with a defined radiant flux generated by the light source 202 is coupled into the light-guiding fiber 203 and guided through the fiber 203 until it is emitted at a predetermined section of the fiber 203. The medical apparatus 201 further comprises a calibration port 204 con-figured to receive the positioning apparatus 100. The calibration port 204 also includes light sensor means (not shown) configured to determine the radiant flux of the laser radiation emerging from the light-guiding fiber 203.

The light source 202 is connected to the light-guiding fiber 201 by way of an appropriate optical unit 205, such that the laser radiation emitted by the laser diodes is coupled into the light-guiding fiber 203.

The medical apparatus 201 further comprises an operating element 206, which may for example be a touch-sensitive display. The operating element 206 allows a user to set one or more parameters of the light emitted from the light source 202, such as output power, irradiation time and/or wavelength. Alternatively, a user may also be able to select from preset protocols of output powers, wavelengths and irradiation times, the protocols each simulating different treatment scenarios. The medical apparatus 201 may be configured to independently ascertain the corresponding operating parameters of the light source 202 that are necessary for a specific treatment set by the user.

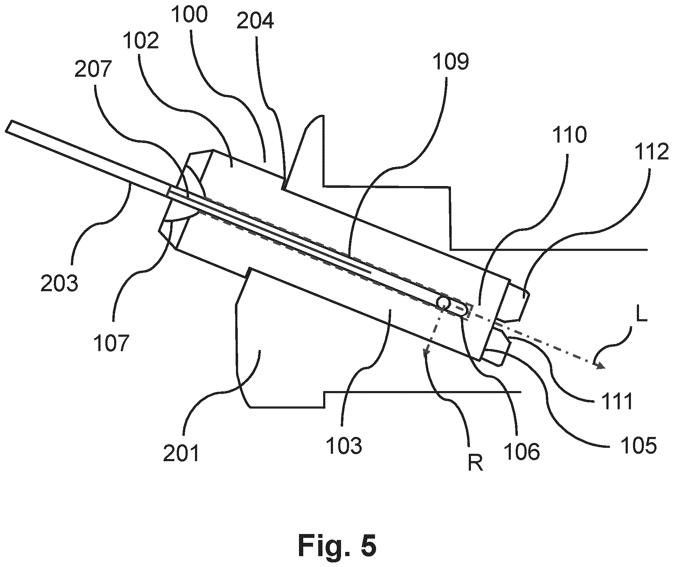

Fig. 5 shows a schematic cross-sectional illustration of a system 200 comprising a medical apparatus 201 and a positioning apparatus 100. In Fig. 5, the light-guiding fiber 203 is already inserted in the positioning apparatus 100 and the positioning apparatus 100 is inserted in the medical apparatus 201. To ensure correct rotational orientation of the fiber 203 in the medical apparatus 201, the fiber bears a visual marker 207 comprising a first strip in a longitudinal direction of the fiber 203 and second strip in circumferential direction around the fiber 203. The control element 107 of the positioning apparatus 100 configured as a groove 114 facilitates correct alignment of the light-guiding fiber 203, since a user can make sure that the visual marker 207 on the fiber 203 is aligned the control element 107. This way, it can be ensured that the light coupled out from the light-guiding fiber 203 at a radial emission point 208 of the fiber 203 will hit the sensor means of the medical apparatus 201.

As is shown in Fig. 5, the channel 106 ends in the elongate portion 103 of the positioning apparatus 100, i.e. it does not fully pass through the main body 101, but rather ends "blindly". Any light that is emitted from the fiber in the longitudinal direction L will pass trough the transparent or semitransparent material of the elongate portion 103 until it passes the second end face 105 of the elongate portion 103, where it finally exits the positioning apparatus 100 through the aperture and frontal cutout 111 in the alignment element 112. Thus, the positioning apparatus 100 of Fig. 5 is configured to transmit light from the light-guiding fiber to a sensor means without the use of any emission opening in the elongate portion of the main body, neither in the radial direction, nor in the longitudinal direction.
light-guiding fiber 203 may be inserted into the channel 106 of positioning apparatus 100 until the fiber 203 abuts against the outer edge of the end section 110 of the channel 106, as the maximum diameter of the light-guiding fiber 203 is bigger than the diameter D2 of the end section 110 of the channel 106.

All features and advantages emerging from the claims, the description and the drawing, including structural details, spatial arrangements and method steps, can be essential to the invention, both on their own and in various combinations.

**Reference numerals**

| | | | |
|---|---|---|---|
| 100 | Positioning apparatus | A | Longitudinal axis |
| 101 | Main body | L | Longitudinal direction |
| 102 | Base portion | R | Radial direction |
| 103 | Elongate portion | D1 | First diameter |
| 104 | First end face | D2 | Second diameter |
| 105 | Second end face | | |
| 106 | Channel | | |
| 107 | Control element | | |
| 109 | Main section | | |
| 110 | End section | | |
| 111 | Frontal cutout | | |
| 112 | Alignment element | | |
| 113 | Aperture | | |
| | | | |
| 200 | System | | |
| 201 | Medical apparatus | | |
| 202 | Light source | | |
| 203 | Light-guiding fiber | | |
| 204 | Calibration port | | |
| 205 | Optical unit | | |
| 206 | Operating element | | |
| 207 | Visual marker | | |

## Claims

1. A positioning apparatus (100) for positioning a light-guiding fiber (203), wherein the positioning apparatus (100) comprises a main body (101) having a base portion (102) and an elongate portion (103), wherein the base portion (102) of the main body (101) has a first end face (104) and wherein the elongate portion (103) of the main body (101) has a second end face (105), and wherein the main body (101) comprises a channel (106) for receiving the light-guiding fiber (203), the channel (106) extending along a longitudinal axis (A) of the main body (101) from the first end face (104) through the base portion (102) and at least partly through the elongate portion (103), **characterized in that** the positioning apparatus (100) is configured to transmit light from a light-guiding fiber (203) to a sensor means without the use of an emission opening in the elongate portion (103) of the main body (101).

2. The positioning apparatus (100) according to claim 1, wherein the elongate portion (103) of the main body (101) or a portion thereof is comprised of a semitransparent material.

3. The positioning apparatus (100) according to claim 1 or claim 2, wherein the elongate portion (103) of the main body (101) or a portion thereof is coated with a semitransparent material.

4. The positioning apparatus (100) according to claim 1, wherein the elongate portion (103) of the main body (101) or a portion thereof is comprised of a fully transparent material.

5. The positioning apparatus (100) according to any of claims 1 to 4, wherein at least a portion of the main body (101) comprises and/or is coated with a sterilizable material.

6. The positioning apparatus (100) according to claim 5, wherein the sterilizable material comprises a plastic, in particular polyoxymethylene.

7. The positioning apparatus (100) according to any of claims 1 to 6, wherein the main body (101) is rotationally symmetric about the longitudinal axis (A) of the main body.

8. The positioning apparatus (100) according to any of claims 1 to 7, wherein base portion (102) of the main body (101) comprises at least one control element (107) for ascertaining the rotational orientation of the light-guiding fiber (203) along the longitudinal axis (A) when the light-guiding fiber (203) is received by the channel (106).

9. The positioning apparatus (100) according to claim 8, wherein the at least one control element (107) comprises one or more of a groove in the first end face (104), a non-symmetric feature of the positioning apparatus (100), a text on the outer surface of the positioning apparatus (100) and a symbol on the outer surface of the positioning apparatus (100).

10. The positioning apparatus (100) according to any of claims 1 to 9, wherein the base portion (102) has a greater radius than the elongate portion (103) of the main body (101).

11. The positioning apparatus (100) according to any of claims 1 to 10, wherein the channel (106) does not penetrate the second end face (105).

12. The positioning apparatus (100) according to any of claims 1 to 11, wherein the channel (106) comprises a main section (109) and an end section (110), and wherein the main section (109) comprises a first diameter (D1) greater than the diameter of the light-guiding fiber (203) and a second diameter (D2) that is less than the diameter of the light-guiding fiber 203).

13. A system (200) comprising the positioning apparatus (100) according to any of claims 1 to 14 and a medical apparatus (201), wherein the medical apparatus (201) comprises at least one light source (202), a calibration port (204), and the sensor means, and wherein the positioning apparatus is configured to position the light-guiding fiber (203) in the calibration port (204) of the medical apparatus (201).

14. The system of claim 15, wherein the medical apparatus (201) comprises at least one lateral photodiode arranged laterally at the calibration port (204).

15. The system according to claim 15 or claim 16, wherein the medical apparatus (201) comprises at least one frontal photodiode arranged at a longitudinal end of the calibration port (204).

16. The system according to any of claims 15 to 17, further comprising at least one light-guiding fiber (203).

17. The system according to claim 18, wherein the light-guiding fiber (203) is configured to emit light from lateral direction of the length of the fiber.

18. The system according to claim 18 or claim 19, wherein the light-guiding fiber (203) is positioned in the calibration port (204) and wherein light emitted by the light-guiding fiber (203) can at least partly emerge from the positioning apparatus (100) to the sensor means.

19. The system according to any of claims 18 to 20, wherein the light-guiding fiber (203) comprises a visual marker (207) for tracing the rotational orientation of the light-guiding fiber (203) when the light-guiding fiber (203) is inserted in the channel (106) of the positioning apparatus (100).

20. The system according to any of claims 15 to 21, wherein the at least one calibration port (204) is configured to receive the positioning apparatus (100) in such a way that, by way of the subsequent insertion of the at least one light-guiding fiber (203) into the positioning apparatus (100), the light-guiding fiber (203) is positioned relative to the sensor means such that the radiant flux of the laser radiation emerging from the light-guiding fiber (203) can be determined by the sensor means.
